# EUROPEAN PATENT SPECIFICATION

(11) **EP 0 456 418 B1**
(45) Date of publication and mention of the grant of the patent: **25.09.1996**
(21) Application number: 91303989.7
(22) Date of filing: 02.05.1991
(51) Int. Cl.: A61K 38/14

(54) **Treatment of clostridium difficile diarrhoea and pseudomembranous colitis**
Behandlung von Clostridium difficile Diarrhöe und pseudomembraner Dickdarmentzündung
Traitement de diarrhée à Clostridium difficile de colite pseudomembranaire

(30) Priority: 07.05.1990 JP 117267/90
(43) Date of publication of application: 13.11.1991
(73) Proprietor: KABUSHIKI KAISHA MIYARISAN SEIBUTSU IGAKU KENKYUSHO, Tokyo (JP)
(72) Inventor: Fujita, Itsuki, Chosei-gun, Chiba-Ken (JP); Taguchi, Nobuhiro, Chosei-gun, Chiba-ken (JP)
(74) Representative: Sheard, Andrew Gregory

(56) References cited:
- CHEMICAL ABSTRACTS, vol. 107, no. 3, 20 July 1987, Columbus, Ohio, US; abstract no. 17606, FUJITA, ITSUKI 'Studies of the anti-diarrheal activity of Clostridium butyricum Miyari II 588' page 55 ;column 2 ;
- BIOLOGICAL ABSTRACTS vol. 86, no. 12 , 1988, Philadelphia, PA, US; abstract no. 122228, TAGUCHI, NOBUHIRO ET AL. 'Prevention of experimental antibiotic- associated diarrhea' page 204 ;

## Description

This invention relates to a preventive and/or curative pharmaceutical composition for Clostridium difficile diarrhoea and pseudomembranous colitis.

The antibacterial agent vancomycin (VCM) is particularly active against Clostridium difficile and has therefore been used in the treatment of Clostridium difficile diarrhoea and pseudomembranous colitis. However, Clostridium difficile is known to form spores in the gastrointestinal tract and these spores are resistant to antibacterial agents and therefore cannot be easily eliminated even by prolonged chemotherapy with vancomycin. Because the spores of Clostridium difficile can survive the therapy it is possible for them to germinate and propagate, resulting in a recurrence of the disease. In addition, it is possible that the prolonged administration of vancomycin to a patient will cause further disturbance of that patient's intestinal microflora which may induce new infections under the disturbed microflora and/or increase the resistance of the pathogenic bacteria to chemicals or other substances.

If vancomycin is administered over a short period of time, further problems occur in that the ratio of survival of the spores is high and consequently, frequent recurrences of the disease induce asthenia in the patient.

The object of the present invention is therefore to overcome the problems in the treatment of Clostridium difficile diarrhoea and pseudomembranous colitis by the short term use of an antibacterial agent such as vancomycin together with an agent which is capable of eliminating surviving spores whilst not obstructing the restoration of intestinal microflora.

In order to achieve this object, the present invention provides a pharmaceutical and/or veterinary composition, comprising cells or spores of a butyric acid bacterium and an antibacterial agent. The antibacterial agent is vancomycin.

The use of such a composition is particularly advantageous because it not only prevents and/or cures the diseases mentioned above but also has extremely low toxicity.

Preferred embodiments of the invention will now be described.

The present inventors have conducted various experiments with the aim of expediting the cure of Clostridium difficile diarrhoea and pseudomembranous colitis and preventing the recurrence of these diseases using a shortened period of administration of an anti-bacterial agent such as vancomycin. The experiments comprise inducing diarrhoea in golden hamsters by the administration of Cefatrizine (CFT), an antibiotic substance generally known as a morbidity model for Clostridium difficile diarrhoea and monitoring the recurrence of the diseases after therapy with vancomycin by observing symptoms of diarrhoea. The affected hamsters were fed with Clostridium butyricum MIYAIRI 588 (CbM), a species of a butyric acid bacteria, as a remedy. The remedy was found to produce highly favourable results.

It was observed that in the recurrence models, when the administration of vancomycin was terminated (after 3 days administration) on improvement of faecal condition, the Clostridium difficle showed positive culture and was detected in the form of spores. Toxicogenic Clostridium difficile and its toxins were detected in the gastrointestinal tracts of the dead animals indicating that the disease had reccurred. Even when administration of vancomycin was continued after improvement of faecal condition (for a total of 10 days), the animals still showed discernible signs of recurrence of the disease within about 5 days although the Clostridium difficile did show negative culture.

In a further experiment, vancomycin was administered to the animals for 3 days and the animals were then fed for 20 days with a feed incorporating ground dry cells of CbM in a proportion such that the animals could ingest approximately 10⁸CFU of the CbM spores per day after which they were fed with a feed containing no CbM. In this experiment, there was a conspicuous decrease in the ratio of recurrence of the diseases and negative culture of Clostridium difficile was attained.

CbM is an anaerobic sporal bacterium which propagates and forms spores in an ordinary culture medium under anaerobic conditions. An example of suitable conditions is a bouillon containing 2% corn starch.

From the results of the experiments it can be concluded that an antibacterial agent such as vancomycin is capable of destroying abnormally over propagated Clostridium difficile. The treatment of the animals with ground dry cells of CbM in a daily dose of about 10⁹CFU/kg is effective in preventing recurrence of the disease.

In the composition of the invention, it is preferred that the amount of vancomycin is in the range of 1mg to 10g and preferably 10mg to 10g and 10⁸CFU of the cells or spores of butyric acid bacterium. The amount of the composition administered to a patient should be in the range of 10⁶ to 10⁹CFU and preferably 10⁶ to 10⁸CFU as cell or spores of butyric acid bacterium per kg of human body weight. The composition is for medical or veterinary use and may be orally administered to patients. The composition may also include a pharmaceutically acceptable carrier.

The term "butyric acid bacterium" refers to a microorganism which yields butyric acid as a product of its metabolism. Clostridium butyricum has been found to be particularly effective in the compositions of the invention.

A major advantage of the compositions of the invention is that they exibit extremely low accute and chronic toxicity.

Specific embodiments of the invention will now be described with reference to the examples.

Clostridium butyricum MIYAIRI 588 used in the experiments has been deposited at Fermentation Research Institute under FERM-BP 2789 and Clostridium difficile Ha.16 has been deposited at the same institute under FERM-BP 2877.

### Example 1

Morbidity models of recurring Clostridium difficile diarrhoea were produced by orally administering to five golden hamsters (60g, male) a suspension of 4.5mg potency of CFT in 0.2ml of purified water with the aid of a catheter. After the CFT treatment, the animals were observed. When any of the animals showed a sign of faecal abnormalities such as diarrhoea or loose passage, a solution of 3mg potency of vancomycin in 0.2ml of purified water was orally administered to that animal six times at 12 hour intervals. All of the treated animals were observed and their rectal faeces were sampled at intervals. The samples were anaerobically cultured for bacterial isolation in a CCFA culture medium (37°C, 72 hours). The isolated Clostridium difficile was placed in a BHI broth and was again cultured (37°C, 48 hours). In the resultant culture broth, the bacteria was tested for toxin-producing ability using a latex reagent (the D-1 toxin of Clostridium difficile can be detected at 500mg/ml (sample)). The diarrhoea faeces of live hamsters and the gastrointestinal tract contents of dead hamsters were also tested for presence or absence of toxin.

Table 1 compares a number of samples and, for each sample shows whether the D-1 toxin of Clostridium difficile was present, and if so, the lethal activity of a toxin on healthy hamsters when the sample was administered to the hamsters in an amount of 1.4 x 10⁸CFU/g.

Table 2 gives an analysis of the biochemical characteristics of Clostridium difficile Ha. 16.

Table 3 shows an analysis of cases in which six doses of vancomycin were administered to the animals and bought about a discernible improvement in the faecal state but was not so effective as to show negative culture of Clostridium difficile. As can be seen from Table 3, all of the animals died of recurrence diarrhoea within 6 to 11 days after the termination of the administration of vancomycin. The limit of detection of Clostridium difficile from faeces was 400CFu/g (faeces).

### Example 2

Animals were treated with long term administration of vancomycin with or without subsequent feeding with CbM. The purpose of this was to attempt to prevent the recurrence of Clostridium difficile diarrhoea and to effect speedy elimination of Clostridium difficile from the interior of the intestinal tract. In the experiment, five animals were treated in the same manner as for example 1 (A group), five animals were subjected to administration of 14 doses of VCM after the end of the treatment of example 1 (B group) and five animals were treated in the same manner as for example 1 and were then allowed to consume for 17 days a feed incorporating ground dry cells of CbM in such a proportion as to allow ingestion of 1 to 5x10⁸CFU of CbM spores per day and thereafter were allowed to consume an ordinary feed containing no CbM (C group). After the treatment, all of the animals were observed and Clostridium difficile was isolated from their intestinal faeces and tested for detection of D-1 toxin. The culture (37°C, 72 hours) for isolation of CbM was anaerobically carried out in a 5% horse blood BL Agar containing 350mg of cycloserine, 1mg of kanamycin, 75 µg of clindamycin and 12mg of trimethoprim per litre of culture medium.

As can be seen from Tables 4 and 5, the B group, when compared with the à group 48 hours after the termination of administration of vancomycin, showed improvement in the faecal state and a discernible decrease of the ratio of Clostridium difficile detection (one out of five animals). In all of the animals under test, the disease recurred after 5 to 8 days follwing the termination of long term administration of vancomycin. This is because, in a gastrointestinal tract which has sub-axenic conditions caused by the long term adminstration of vancomycin, a small number of spores which would normally be prevented from propagation by the intestinal microflora are allowed to germinate and propagate. This effect results in the recurrence of the disease in the B group. In contrast, in the C group, although the recurrence of the disease was observed in two of the five animals, negative culture of Clostridium difficile was finally attained in the remainder of the animals, representing a survival rate of 60%. It can therefore be seen that the therapy comprising short term administration of vancomycin together with a continuous administration of CbM was effective in preventing germination and propagation of Clostridium difficile in the intestine without imparing the intestinal microflora and thereby obtaining complete cure of the disease including prevention of the recurrence of the disease.

## Claims (Claims for the following Contracting State(s): CH, DE, FR, GB, IT, LI)

1. A pharmaceutical and/or veterinary composition comprising cells or spores of a butyric acid bacterium and vancomycin.

2. A composition according to claim 1, wherein the butyric acid bacterium is Clostridium butyricum.

3. A composition according to claim 1 or claim 2, wherein the amount of vancomycin is in the range of 1mg to 10g per 10⁸CFU of the cells or spores of the butyric acid bacterium.

4. The use of cells or spores of a butyric acid bacterium and an antibacterial agent, in the preparation of an agent for the prophylaxis and/or treatment of Clostridium difficile diarrhoea and pseudomembranous colitis.

5. The use according to claim 4, wherein the antibacterial agent is vancomycin.

6. The use according to claim 4 or claim 5, wherein the butyric acid bacterium is Clostridium butyricum.

7. The use according to any one of claims 4 to 6, wherein the amount of vancomycin is in the range of 1mg to 10g per 10⁸CFU of the cells or spores of the butyric acid bacterium.

8. A product comprising cells or spores of a butyric acid bacterium and vancomycin, as a combined preparation for simultaneous, separate or sequential administration.

## Claims (Claims for the following Contracting State(s): ES)

1. A process for the preparation of a pharmaceutical and/or veterinary composition, the process comprising admixing cells or spores of a butyric acid bacterium and vancomycin.

2. A process according to claim 1, wherein the butyric acid bacterium is Clostridium butyricum.

3. A process according to claim 1 or claim 2, wherein the amount of the antibacterial agent is in the range of 1mg to 10g per 10⁸CFU of the cells or spores of the butyric acid bacterium.

4. The use of cells or spores of a butyric acid bacterium and an antibacterial agent, in the preparation of an agent for the prophylaxis and/or treatment of Clostridium difficile diarrhoea and pseudomembranous colitis.

5. The use according to claim 4, wherein the antibacterial agent is vancomycin.

6. The use according to claim 4 or claim 5, wherein the butyric acid bacterium is Clostridium butyricum.

7. The use according to any one of claims 4 to 6, wherein the amount of vancomycin is in the range of 1mg to 10g per 10⁸CFU of the cells or spores of the butyric acid bacterium.

8. A product comprising cells or spores of a butyric acid bacterium and vancomycin, as a combined preparation for simultaneous, separate or sequential administration.

## Patentansprüche (Patentansprüche für folgende(n) Vertragsstaat(en): CH, DE, FR, GB, IT, LI)

1. Pharmazeutische und/oder tierärztliche Zusammensetzung, die Zellen oder Sporen eines Buttersäure-Bakteriums und Vancomycin aufweist.

2. Zusammensetzung gemäß Anspruch 1, dadurch gekennzeichnet, daß das Buttersäure-Bakterium Clostridium butyricum ist.

3. Zusammensetzung gemäß Anspruch 1 oder Anspruch 2, dadurch gekennzeichnet, daß die Menge an Vancomycin im Bereich von 1 mg bis 10 g pro 10⁸ CFU der Zellen oder Sporen des Buttersäure-Bakteriums liegt.

4. Verwendung von Zellen oder Sporen eines Buttersäure-Bakteriums und einem antibakteriellen Mittel zur Herstellung eines Mittels für die Prophylaxe und/oder Behandlung von Clostridium difficile diarrhoea und pseudomembranöser Kolitis.

5. Verwendung gemäß Anspruch 4, dadurch gekennzeichnet, daß das antibakterielle Mittel Vancomycin ist.

6. Verwendung gemäß Anspruch 4 oder Anspruch 5, dadurch gekennzeichnet, daß das Buttersäure-Bakterium Clostridium butyricum ist.

7. Verwendung gemäß einem der Ansprüche 4 bis 6, dadurch gekennzeichnet, daß die Menge an Vancomycin im Bereich von 1 mg bis 10 g pro 10⁸ CFU der Zellen oder Sporen des Buttersäure-Bakteriums liegt.

8. Produkt, das Zellen oder Sporen eines Buttersäure-Bakteriums und Vancomycin aufweist, als ein kombiniertes Präparat zur simultanen, separaten oder sequentiellen Varabreichung.

## Patentansprüche (Patentansprüche für folgende(n) Vertragsstaat(en): ES)

1. Verfahren zur Herstellung einer pharmazeutischen und/oder tierärztlichen Zusammensetzung, wobei das Verfahren das Beimischen von Zellen oder Sporen eines Buttersäure-Bakteriums und Vancomycin beinhaltet.

2. Verfahren gemäß Anspruch 1, dadurch gekennzeichnet, daß das Buttersäure-Bakterium Clostridium butyricum ist.

3. Verfahren gemäß Anspruch 1 oder Anspruch 2, dadurch gekennzeichnet, daß die Menge an Vancomycin im Bereich von 1 mg bis 10 g pro 10⁸ CFU der Zellen oder Sporen des Buttersäure-Bakteriums liegt.

4. Verwendung von Zellen oder Sporen eines Buttersäure-Bakteriums und einem antibakteriellen Mittel zur Herstellung eines Mittels für die Prophylaxe und/oder Behandlung von Clostridium difficile diarrhoea und pseudomembranöser Kolitis.

5. Verwendung gemäß Anspruch 4, dadurch gekennzeichnet, daß das antibakterielle Mittel Vancomycin ist.

6. Verwendung gemäß Anspruch 4 oder Anspruch 5, dadurch gekennzeichnet, daß das Buttersäure-Bakterium Clostridium butyricum ist.

7. Verwendung gemäß einem der Anspruch 4 bis 6, dadurch gekennzeichnet, daß die Menge an Vancomycin im Bereich von 1 mg bis 10 g pro 10⁸ CFU der Zellen oder Sporen des Buttersäure-Bakteriums liegt.

8. Produkt, das Zellen oder Sporen eines Buttersäure-Bakteriums und Vancomycin aufweist, als ein kombiniertes Präparat zur simultanen. separaten oder sequentiellen Verabreichung.

## Revendications (Revendications pour l'(les) Etat(s) contractant(s) suivant(s): CH, DE, FR, GB, IT, LI)

1. Composition pharmaceutique et/ou vétérinaire comprenant des cellules ou des spores d'une bactérie à acide butyrique et de la vancomycine.

2. Composition selon la revendication 1, dans laquelle la bactérie à acide butyrique est Clostridium butyricum.

3. Composition selon la revendication 1 ou la revendication 2, dans laquelle la quantité de vancomycine est dans le domaine de 1 mg à 10 g pour 10⁸ UFC de cellules ou de spores de la bactérie à acide butyrique.

4. Utilisation de cellules ou de spores d'une bactérie à acide butyrique et d'un agent antibactérien dans la préparation d'un agent pour la prophylaxie et/ou le traitement de la diarrhée à Clostridium difficile et de la colite pseudomemoraneuse.

5. Utilisation selon la revendication 4, dans laquelle l'agent antibactérien est la vancomycine.

6. Utilisation selon la revendication 4 ou la revendication 5, dans laquelle la bactérie à acide butyrique est Clostridium butyricum.

7. Utilisation selon l'une quelconque des revendications 4 à 6, dans laquelle la quantité de vancomycine est dans le domaine de 1 mg à 10 g pour 10⁸ UFC de cellules ou de spores de la bactérie à acide butyrique.

8. Produit comprenant des cellules ou des spores d'une bactérie à acide butyrique et de la vancomycine, sous forme d'une préparation cominée pour l'administration simultanée, séparée ou consécutive.

## Revendications (Revendications pour l'(les) Etat(s) contractant(s) suivant(s): ES)

1. Procédé pour la préparation d'une composition pharmaceutique et/ou vétérinaire, le procédé comprenant le mélange de cellules ou de spores d'une bactérie à acide butyrique et de vancomycine.

2. Procédé selon la revendication 1, dans lequel la bactérie à acide butyrique est Clostridium butyricum.

3. Procédé selon la revendication 1 ou la revendication 2, dans lequel la quantité d'agent antibactérien est dans le domaine de 1 mg à 10 g pour 10⁸ UFC de cellules ou de spores de la bactérie à acide butyrique.

4. Utilisation de cellules ou de spores d'une bactérie à acide butyrique et d'un agent antibactérien dans la préparation d'un agent pour la prophylaxie et/ou le traitement de la diarrhée à Clostridium difficile et de la colite pseudomembraneuse.

5. Utilisation selon la revendication 4, dans laquelle l'agent antibactérien est la vancomycine.

6. Utilisation selon la revendication 4 ou la revendication 5, dans laquelle la bactérie à acide butyrique est Clostridium butyricum.

7. Utilisation selon l'une quelconque des revendications 4 à 6, dans laquelle la quantité de vancomycine est dans le domaine de 1 mg à 10 g pour 10⁸ UFC de cellules ou de spores de la bactérie à acide butyrique.

8. Produit comprenant des cellules ou des spores d'une bactérie à acide butyrique et de la vancomycine, sous forme d'une préparation combinée pour l'administration simultanée, séparée ou consécutive.
